# EUROPÄISCHE PATENTANMELDUNG

(11) **EP 2 191 868 A1**
(43) Veröffentlichungstag der Anmeldung: **02.06.2010**
(21) Anmeldenummer: 08020704.6
(22) Anmeldetag: 28.11.2008
(51) Int. Cl.: A61Q 15/00, A61K 8/81

(54) **Ethylenoxid-freie Antiperspirant/Desodorant-Zubereitungen**

(71) Anmelder: Cognis IP Management GmbH, 40589 Düsseldorf (DE)
(72) Erfinder: Suess, Ev, 40213 Düsseldorf (DE); Milardovic, Jadranka, 40235 Düsseldorf (DE); Schulte, Petra, 50733 Köln (DE); Strauss, Gabriele, 40589 Düsseldorf (DE)
(74) Vertreter: Gittinger, Andreas

(57) **Zusammenfassung**

Die Anmeldung betrifft Ethylenoxid-freie Antiperspirant/Desodorant-Zubereitungen, enthaltend einen Antiperspirant-/Desodorant-Wirkstoff und ein quaternäres Polymer mit der INCI-Bezeichnung Polyquaternium 37.

## Beschreibung

### Gebiet der Erfindung

Die vorliegende Erfindung betrifft Ethylenoxid-freie Antiperspirant/Desodorant-Zubereitungen auf Basis des quatemären kationischen Polymers mit der INCI-Bezeichnung Polyquaternium 37.

### Stand der Technik

AP/Deo-Formulierungen des Marktes sind in der Regel Ethylenoxid-haltig, da die in den Formulierungen verwendeten Emulgatoren üblicherweise Ethylenoxid (EO) enthalten, was aus ökologischer Sicht kritisch gesehen wird, auch wenn sie hinsichtlich der Schutzwirkung gegen Schweißgeruch eine ausreichende Leistung haben.

Es gibt auch EO-fteie AP/Deo Formulierungen auf dem Markt, welche in der Regel auf Glycerylstearatcitrat basieren und weder durch die Stabilität noch durch ihre sensorische Performance überzeugen können, da sie mit einer starken Klebrigkeit einhergehen.

Es gibt zwar Lösungsansätze in der Literatur, um ein trockenes Endgefühl zu erreichen. Diese beziehen sich jedoch ausschließlich auf wasserfreie Systeme (W. Umbach, Kosmetik und Hygiene, Wiley-VCH, 3. Auflage, S. 371), die zwar richtigerweise ein trockenes Hautgefühl nach dem Auftragen aufweisen, was aber nach kurzer Zeitz stumpf und unangenehm erscheint.

Aufgabe der vorliegenden Erfindung war es, AP/Deo-Formullerungen zur Verfügung zu stellen, die zum einen eine gute Stabilität aufweisen und zum anderen direkt nach Anwendung in die Haut einziehen und die ein trockenes, nicht klebriges Endgefühl hinterlassen, ohne dass die Schutzwirkung reduziert wird. Weiterhin sollte auf die ökologisch bedenklichen Ethylenoxide verzichtet werden können.

### Beschreibung der Erfindung

Gegenstand der vorliegenden Erfindung sind Ethylenoxid-freie Antiperspirant/Desodorant-Zubereitungen mit folgenden Bestandteilen:
a. Antiperspirant-/Desodorant-Wirkstoff,
b. einem quaternären Polymer mit der INCI-Bezeichnung Polyquatemium 37.

In einer bevorzugten Ausführungsform enthalten die Zubereitungen:
a. Antiperspirant-/Desodorant-Wirkstoff,
b. ein quaternäres Polymer mit der INCI-Bezeichnung Polyquaternium 37, und
c. eine lipophile Phase, die aus flüssigem Öl und/oder Wachs gebildet ist.

Bevorzugt sind Antiperspirant/Desodorant-Zubereitungen, welche die Bestandteile in folgenden Anteilen enthalten:
a. im allgemeinen 1- 30 Gew.-%, bevorzugt 5 - 20 Gew.-% und ganz besonders bevorzugt 10 -15 Gew.-% des Antiperspirant-/Desodorant-Wirkstoffs
b. im Allgemeinen 0,1-10 Gew.-% und bevorzugt 1 - 5 Gew.-% eines quatemären Polymers mit der unten genannten Strukturformel und der INCI Bezeichnung Polyquaternium 37, und
c. im allgemeinen 1 - 20 Gew.-% und idealer Weise 5 - 15 Gew.-% der lipophilen Phase, jeweils bezogen auf das Gesamtgewicht der Antiperspirant/Desodorant-Zubereitung.

Bei Polyquaternium 37 handelt es sich um ein quaternäres Polymer, welches der folgenden allgemeinen Formel entspricht:

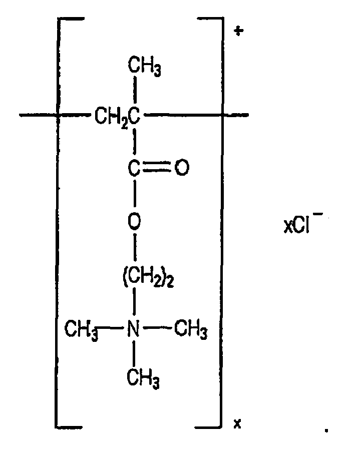

Überraschenderweise wurde gefunden, dass Ethylenoxid-freie Antiperspirant/Desodorant-Zubereitungen mit verbesserter Stabilität hergestellt werden können, wenn die Zubereitungen das quaternäre Polymer mit der INCI-Bezeichnung Polyquaternium 37 enthalten. In einer bevorzugten Ausführungsform sind die Zubereitungen ganz Emulgator-frei und enthalten eine lipophile Phase. Diese Zubereitungen zeichnen sich zudem dadurch aus, dass sie zusätzlich eine deutlich bessere Sensorik aufweisen. Insbesondere kann mit Polyquaternium 37 das klebrige, stumpfe und wachsige Hautgefühl bei der Anwendung, das bei Zubereitungen des Stands der Technik gegeben ist, z. B. Zubereitungen, die Glycerylstearatcitrat enthalten, vermieden werden oder zumindest deutlich vermindert werden. Bei alledem wird die Schutzleistung, also die Wirkung als Antitranspirans/Deodorant, beibehalten. Handelsübliche und erfindungsgemäß verwendbare Polyquaternium 37 Typen sind z.B.: Ultragel^{®} 300 - Cognis GmbH, OriStar PQ37 - Orient Stars LLC, Synthalen CN - 3V Group, Synthalen CR - 3V Group, Synthalen CU - 3V Group, Syntran PC 5320 - Interpolymer Corporation

Bevorzugt wird daher eine AP/DEO Zubereitung, die das Polyquaternium 37 im Allgemeinen in einem Anteil von 0,1 - 10 Gew.-%, bezogen auf das Gesamtgewicht der Antipersplrant/Desodorant-Zubereitung, enthält, idealer Weise 1 bis 5 Gew.-%.

### Emulgator

Üblicherweise enthalten AP/Deo-Formulierungen Ethylenoxid-haltige Emulgatoren. Ethylenoxid ist aber, wie eingangs erwähnt, ökologisch bedenklich, und es besteht daher das Bestreben, dieses in kosmetischen Zubereitungen zu vermeiden. Erfindungsgemäß ist in den AP/Deo-Zubereitungen kein Ethylenoxid enthalten. In einer bevorzugten Ausführungsform ist in der erfindungsgemäßen AP/Deo-Zubereitungen kein Emulgator enthalten. In anderen Ausführungsformen der vorliegenden Erfindung kann ein Emulgator und ggf. Coemulgator in den AP/Deo-Zubereitungen enthalten sein, solange dieser und die ganze Formulierung kein Ethylenoxid enthält. Wenn ein solcher Emulgator enthalten ist, kann es sich dabei um die üblicherweise in kosmetischen Zubereitungen verwendeten Emulgatoren handeln, die auch in üblichen Mengen eingesetzt werden. Für Beispiele der verwendbaren Emulgatoren und Coemulgatoren wird auf die WO 2008/019773 verwiesen, mit der Vorgabe, dass es sich nicht um Ethylenoxid-haltige Emulgatoren und Coemulgatoren handelt.

### Antiperspirant/Desodorant-Wirkstoff

Erfindungsgemäß sind als Antiperspirant/Desodorant-Wirkstoff alle Wirkstoffe geeignet, die Körpergerüchen entgegen wirken, diese überdecken oder beseitigen. Körpergerüche entstehen durch die Einwirkung von Hautbakterien auf apokrinen Schweiß, wobei unangenehm riechende Abbauprodukte gebildet werden. Als Antiperspirant /Desodorant Wirkstoffe eignen sich insbesondere Verbindungen ausgewählt aus der Gruppe bestehend Antiperspirantien, Esteraseinhibitoren, bakterizide bzw. bakteriostatische Wirkstoffe und/oder schweißabsorbierende Substanzen.

Der Antiperspirant/Desodorant-Wirkstoff oder die Antiperspirant/Desodorant-Wirkstoffe ist/sind in üblicherweise verwendeten Mengen enthalten, bevorzugt in einem Anteil von 1 bis 30 Gew.-%, bezogen auf das Gesamtgewicht der Antiperspirant/Desodorant-Zubereitung, bevorzugter 5 bis 20 Gew.-% und noch bevorzugter 10 bis 15 Gew.-%. Es kann ein einziger Antiperspirant/Desodorant-Wirkstoff oder es können mehrere Antiperspirant/Desodorant-Wirkstoffe enthalten sein. Im letzteren Fall beziehen sich die obigen Prozentangaben auf die Gesamtmenge der enthaltenen Antiperspirant/Desodorant-Wirkstoffe.

### Antiperspirantien

Bei Antiperspirantien handelt es sich um Salze des Aluminiums, Zirkoniums oder des Zinks. Solche geeigneten antihydrotisch wirksamen Wirkstoffe sind z.B. Aluminiumchlorid, Aluminiumchlorhydrat, Aluminiumdichlorhydrat, Aluminiumsesquichlorhydrat und deren Komplexverbindungen z. B. mit Propylenglycol-1,2. Aluminiumhydroxyallantoinat, Aluminiumchioridtartrat, Aluminium-Zirkonium-Trichlorohydrat, Aluminium-Zirkonium-tetrachlorohydrat, Aluminium-Zirkonium-pentachlorohydrat und deren Komplexverbindungen z. B. mit Aminosäuren wie Glycin. Vorzugsweise werden Aluminiumchlorhydrat, Aluminium-Zirkonium-tetrachlorohydrat, Aluminium-Zirkonium-pentachlorhydrat und deren Komplexverbindungen eingesetzt, wie z.B. Locron L oder ACH-303 50% Solution, Rezal 67 oder AZ-7373 Powder, Rezal 36 GC oder AZG-7226 50% Solution.

Die erfindungsgemäßen Zubereitungen können die Antiperspirantien in Mengen von 1 bis 20, vorzugsweise 5 bis 20 und insbesondere 8 bis 20 Gew.-% - bezogen auf das Gesamtgewicht der Antiperspirant-/Desodorant-Zubereitung - enthalten.

### Esteraseinhibitoren

Beim Vorhandensein von Schweiß im Achselbereich werden durch Bakterien extrazelluläre Enzyme - Esterasen, vorzugsweise Proteasen und/oder Lipasen - gebildet, die im Schweiß enthaltene Ester spalten und dadurch Geruchsstoffe freisetzen. Als Esteraseinhibitoren geeignet sind vorzugsweise Trialkylcitrate wie Trimethylcitrat, Tripropylcitrat, Triisopropylcitrat, Tributylcitrat und insbesondere Triethylcitrat (Hydagen® CAT, Cognis GmbH, Düsseldod/FRG). Die Stoffe inhibieren die Enzymaktivität und reduzieren dadurch die Geruchsbildung. Weitere Stoffe, die als Esteraseinhibitoren in Betracht kommen, sind Sterolsulfate oder -phosphate, wie beispielsweise Lanosterin-, Cholesterin-, Campesterin-, Stigmasterin- und Sitosterinsulfat bzw -phosphat, Dicarbonsäuren und deren Ester, wie beispielsweise Glutarsäure, Glutarsäuremonoethylester, Glutarsäurediethylester, Adipinsäure, Adipinsäuremonoethylester, Adipinsäurediethylester, Malonsäure und Malonsäure-diethylester, Hydroxycarbonsäuren und deren Ester wie beispielsweise Citronensäure, Äpfelsäure, Weinsäure oder Weinsäurediethylester sowie Zinkglycinat.

Die erfindungsgemäßen Zubereitungen können die Esteraseinhibitoren in Mengen von 0,01 bis 20, vorzugsweise 0,1 bis 10 und insbesondere 0,3 bis 5 Gew.-% - bezogen auf das Gesamtgewicht der Antiperspirant-/Desodorant-Zubereitung - enthalten.

### Bakterizide bzw. bakteriostatische Wirkstoffe

Typische Beispiele für geeignete bakterizide bzw. bakteriostatische Wirkstoffe sind insbesondere Chitosan und Phenoxyethanol. Besonders wirkungsvoll hat sich auch 5-Chlor-2-(2,4-dichlorphenoxy)-phenol erwiesen, das unter der Marke Irgasan® von der Ciba-Geigy, Basel/CH vertrieben wird. Als keimhemmende Mittel sind grundsätzlich alle gegen grampositive Bakterien wirksamen Stoffe geeignet, wie z. B. 4-Hydroxybenzoesäure und ihre Salze und Ester, N-(4-Chlorphenyl)-N'-(3,4 dichlor phenyl)hamstoff, 2,4,4'-Trichlor-2'-hydroxydiphenylether (Triclosan), 4-Chlor-3,5-dimethylphenol, 2,2'-Methylen-bis(6-brom-4-chlorphenol), 3-Methyl-4-(1-methylethyl)phenol, 2-Benzyl-4-chlorphenol, 3-(4-Chlorphenoxy)-1,2-propandiol, 3-lod-2-propinylbutylcarbamat, Chlorhexidin, 3,4,4'-Trichlorcarbanilid (TTC), antibakterielle Riechstoffe, Thymol, Thymianöl, Eugenol, Nelkenöl, Menthol, Minzöl, Farnesol, Phenoxyethanol, Glycerinmonocaprinat, Glycerinmonocaprylat, Glycerinmonolaurat (GML), Diglycerinmonocaprinat (DMC), Salicylsäure-N-alkylamide wie z. B. Salicylsäure-n-octylamid oder Salicylsäure-n-decylamid.

Die erfindungsgemäßen Zubereitungen können die bakteriziden bzw. bakteriostatischen Wirkstoffe in Mengen von 0,01 bis 5 und vorzugsweise 0,1 bis 2 Gew.-% - bezogen auf das Gesamtgewicht der Antiperspirant-/Desodorant-Zubereitung - enthalten.

### Schweißabsorbierende Substanzen

Als schweißabsorbierende Substanzen kommen modifizierte Stärke, wie z.B. Dry Flo Plus (Fa. National Starch), Silikate, Talkum und andere Substanzen ähnlicher Modifikation, die zur Schweißabsorption geeignet erscheinen. Die erfindungsgemäßen Zubereitungen können die schweißabsorbierende Substanzen in Mengen von 0,1 bis 20, vorzugsweise 1 bis 20 und insbesondere 2 bis 8 Gel.-% - bezogen auf das Gesamtgewicht der Antiperspirant-/Desodorant-Zubereitung - enthalten.

### Lipophile Phase

Die lipophile Phase ist erfindungsgemäß aus Wachs(en) und/oder flüssigen Ölen gebildet oder besteht daraus. Bevorzugt ist eine Kombination von Ölen und Wachsen.
Die lipophile Phase ist bevorzugt in einem Anteil von 1 - 20 Gew.-% bezogen auf das Gesamtgewicht der Antiperspirant-/Desodorant-Zubereitung, enthalten, idealer Weise 5 -15 Gew.-%.

Unter dem Begriff **"Öle"** (synonym verwendet: Ölkomponente) werden wasserunlösliche, bei 30°C flüssige, organische Verbindungen mit relativ niedrigem Dampfdruck bezeichnet. Das gemeinsame Merkmal der Öle ist nicht ihre übereinstimmende chemische Konstitution, sondere ihre ähnliche physikalische Konsistenz.

Als Ölkomponenten sind beispielsweise die nachstehend genannten Verbindungsklassen geeignet, soweit diese bei 30°C flüssig sind. So z.B. Guerbetalkohole auf Basis von Fettalkoholen mit 6 bis 18, vorzugsweise 8 bis 10 Kohlenstoffatomen (z. B. Eutanol® G) , Ester von linearen C₆-C₂₂-Fettsäuren mit linearen oder verzweigten C₆-C₂₂-Fettalkoholen (z.B. Cetiol® Sensoft) bzw. Ester von verzweigten C₆-C₁₃-Carbonsäuren mit linearen oder verzweigten C₆-C₂₂-Fettalkoholen, wie z.B. Myristylmyristat, Myristylpalmitat, Myristylstearat, Myristylisostearat, Myrisiyloleat, Myristylbehenat, Myristylerucat, Cetylmyristat, Cetylpalmitat, Cetylstearat, Cetylisostearat, Cetyloleat, Cetylbehenat, Cetylerucat, Stearylmyristat, Stearylpalmitat, Stearylstearat, Stearylisostearat, Stearyloleat, Stearylbehenat, Stearylerucat, Isostearylmyristat, Isostearylpalmitat, Isostearylstearat, lsostearylisostearat, Isostearyloleat, Isostearylbehenat, Isostearyloleat, Oleylmyristat, Oleylpalmitat, Oleylstearat, Oleylisostearat, Oleyloleat, Oleylbehenat, Oleylerucat, Behenylmyristat, Behenylpalmitat, Behenylstearat, Behenylisostearat, Behenyloleat, Behenylbehenat, Behenylerucat, Erucylmyristat, Erucylpalmitat, Erucylstearat, Erucylisostearat, Erucyloleat, Erucylbehenat, Erucylerucat und Hexyldecylstearat (Eutanol® G 16 S). Daneben eignen sich Ester von linearen C₆-C₂₂-Fettsäuren mit verzweigten Alkoholen, insbesondere 2- Ethylhexanol, Ester von C₃-C₃₈-Alkylhydroxycarbonsäuren mit linearen oder verzweigten C₆-C₂₂-Feftalkoholen - insbesondere Dioctylmalat -, Ester von linearen und/oder verzweigten Fettsäuren mit mehrwertigen Alkoholen (wie z.B. Propylenglycol, Dimerdiol oder Trimertriol) und/oder Guerbetalkoholen, Triglyceride auf Basis C₆-C₁₀-Fettsäuren, flüssige Mono-/Di-/Triglyceridmischungen auf Basis von C₆-C₁₈-Fettsäuren, Ester von C₆-C₂₂-Fettalkoholen und/oder Guerbetalkoholen mit aromatischen Carbonsäuren, insbesondere Benzoesäure, Ester von C₂-C₁₂-Dicarbonsäuren mit linearen oder verzweigten Alkoholen mit 1 bis 22 Kohlenstoffatomen oder Polyolen mit 2 bis 10 Kohlenstoffatomen und 2 bis 6 Hydroxylgruppen, pflanzliche Öle, verzweigte primäre Alkohole, substituierte Cyclohexane wie z.B. 1 ,3-Dialkylcyclohexane, lineare und verzweigte C₆-C₂₂-Fettalkoholcarbonate, wie z.B. Dicaprylyl Carbonate (Cetiol® CC), Guerbetcarbonate auf Basis von Fettalkoholen mit 6 bis 18, vorzugsweise 8 bis 10 C Atomen, Ester der Benzoesäure mit linearen und/oder verzweigten C₆-C₂₂-Alkoholen (z.B. Finsolv® TN), lineare oder verzweigte, symmetrische oder unsymmetrische Dialkylether mit 6 bis 22 Kohlenstoffatomen pro Alkylgruppe, wie z.B. Dicaprylyl Ether (Cetiol® OE), Ringöffnungsprodukte von epoxidierten Fettsäureestern mit Polyolen (Hydagen® HSP, Sovermol® 750, Sovermol® 1102), Siliconöle (Cyclomethicone, Siliciummethicontypen u.a. und/oder aliphatische bzw. naphthenische Kohlenwasserstoffe, wie z.B. wie Mineralöl, Vaseline, Petrolatum, Squalan, Squalen oder Dialkylcyclohexane.

Als weitere Ölkörper kommen beispielsweise Silikonöle in Frage. Sie können als cyclische und/oder lineare Silikonöle vorliegen. Silikonöle sind hochmolekulare synthetische polymere Verbindungen, in denen Silicium- Atome über Sauerstoff-Atome ketten-und/oder netzartig verknüpft und die restlichen Valenzen des Siliciums durch Kohlenwasserstoff-Reste (meist Methyl-, seltener Ethyl-, Propyl-, PhenylGruppen u. a.) abgesättigt sind. Systematisch werden die Silikonöle als Polyorganosiloxane bezeichnet. Die methylsubstituierten Polyorganosiloxane, welche die mengenmäßig bedeutendsten Verbindungen dieser Gruppe darstellen und sich durch die folgende Strukturformel 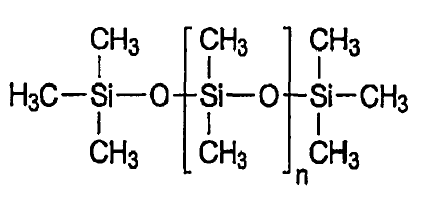 auszeichnen werden auch als Polydimethylsiloxan bzw. Dimethicon (INCI) bezeichnet. Dimethicone gibt es in verschiedenen Kettenlängen bzw. mit verschiedenen Molekulargewichten.

Vorteilhafte Polyorganosiloxane im Sinne der vorliegenden Erfindung sind beispielsweise Dimethylpolysiloxan [Poly (dimethylsiloxan)], welche beispielsweise unter den Handels- bezeichnungen Abil 10 bis 10 000 bei Evonik Goldschmidt erhältlich sind. Ferner vorteilhaft sind Phenylmethylpolysiloxan (INCI: Phenyl Dimethicon, Phenyl Trimethicon), cyclische Silikone (Octamethylcyclotetrasiloxan bzw. Decamethylcyclopentasiloxan), welche nach INCI auch als Cyclomethicon bezeichnet werden, aminomodifizierte Silikone (INCI: Amodimethicone) und Silikonwachse, z. B. Polysiloxan-Polyalkylen-Copolymere (INCI: Stearyl Dimethicon und Cetyl Dimethicon) und Dialkoxydimethylpolysiloxane (Stearoxy Dimethicon und Behenoxy Stearyl Dimethicon), welche als verschiedene Abil-Wax-Typen bei Evonik Goldschmidt erhältlich sind. Aber auch andere Silikonöle sind vorteilhaft im Sinne der vorliegenden Erfindung zu verwenden, beispielsweise Cetyidimethicon, Hexamethylcyclo-trisiloxan, Polydimethylsiloxan, Poly (methylphenylsiloxan). Erfindungsgemäß besonders bevorzugte Silikone sind Dimethicon und Cyclomethicon.

Als Ölkomponenten eignen sich auch Polycarbonate, wie beispielsweise in WO 03/041676 beschrieben, auf die hier ausdrücklich Bezug genommen wird.

Als Polycarbonate besonders geeignet ist das unter der INCI Bezeichnung Hydrogenated Dimer Dilinoleyl / Dimethylcarbonate Copolymer, welches als Handelsprodukt Cosmedia® DC von Cognis GmbH erhältlich ist.

Die Dialkylcarbonate und Dialkylether können symmetrisch oder unsymmetrisch, verzweigt oder unverzweigt, gesättigt oder ungesättigt sein und lassen sich nach Reaktionen, die aus dem Stand der Technik hinlänglich bekannt sind, herstellen.

Erfindungsgemäß einsetzbar sind u.a. auch Kohlenwasserstoffe, vorzugsweise mit einer Kettenlänge 8 bis 40 C-Atomen. Sie können verzweigt oder unverzweigt sein, gesättigt oder ungesättigt. Unter diesen sind verzweigte, gesättigte C₈-C₄₀-Alkane bevorzugt. Es können sowohl Reinsubstanzen eingesetzt werden als auch Substanzgemische. Üblicherweise handelt es sich um Substanzgemische verschiedener isomerer Verbindungen. Zusammensetzungen, die Alkane mit 10 bis 30, vorzugsweise 12 bis 20, und besonders bevorzugt 16 bis 20 Kohlenstoffatome aufweisen, sind besonders geeignet, und unter diesen ein Gemisch aus Alkanen, welches wenigstens 10 Gew.-% verzweigte Alkane bezogen auf die Gesamtmenge der Alkane enthält. Vorzugsweise handelt es sich um verzweigte, gesättigte Alkane. Besonders gut geeignet sind Gemische aus Alkanen, welche mehr als 1 Gew.-% 5,8-Diethyldodecan und/oder mehr als 1 Gew.-% Didecen enthalten.

Es kann erfindungsgemäß eine Öl oder ein Gemisch aus mehreren Ölkomponenten verwendet werden.

Unter dem Begriff **Wachs** (synonym verwendet: Wachskomponente) werden üblicherweise alle natürlichen oder künstlich gewonnenen Stoffe und Stoffgemische mit folgenden Eigenschaften verstanden: sie sind von fester bis brüchig harter Konsistenz, grob bis feinteilig, durchscheinend bis trüb und schmelzen oberhalb von 30°C ohne Zersetzung. Sie sind schon wenig oberhalb des Schmelzpunktes niedrigviskos und nicht fadenziehend und zeigen eine stark temperaturabhängige Konsistenz und Löslichkeit. Erfindungsgemäß einsetzbar ist eine Wachskomponente oder ein Gemisch von Wachskomponenten, die bei 30°C oder darüber schmelzen.

Als Wachse können erfindungsgemäß auch Fette und fettähnliche Substanzen mit wachsartiger Konsistenz eingesetzt werden, solange sie den geforderten Schmelzpunkt haben. Hierzu gehören u.a. Fette (Triglyceride), sowie natürliche und synthetische Wachse oder beliebige Gemische dieser Substanzen.

Unter Fetten versteht man Triacylglycerine, also die Dreifachester von Fettsäuren mit Glycerin. Bevorzugt enthalten sie gesättigte, unverzweigte und unsubstituierte Fettsäurereste. Hierbei kann es sich auch um Mischester, also um Dreifachester aus Glycerin mit verschiedenen Fettsäuren handeln. Erfindungsgemäß einsetzbar sind so genannte gehärtete Fette und Öle, die durch Partialhydrierung gewonnen werden. Pflanzliche gehärtete Fette und Öle sind bevorzugt, z. B. gehärtetes Rizinusöl, Erdnussöl, Sojaöl, Rapsöl, Rübsamenöl, Baumwollsaatöl, Sojaöl, Sonnenblumenöl, Palmöl, Palmkernöl, Leinöl, Mandelöl, Maisöl, Olivenöl, Sesamöl, Kakaobutter und Kokosfett.

Geeignet sind u.a. die Dreifachester von Glycerin mit C₁₂-C₆₀-Fettsäuren und insbesondere C₁₂-C₃₆-Fettsäuren. Hierzu zählt gehärtetes Rizinusöl, ein Dreifachester aus Glycerin und einer Hydroxystearinsäure, der beispielsweise unter der Bezeichnung Cutina® HR im Handel ist. Ebenso geeignet sind Glycerintristearat, Glycerintribehenat (z. B. Syncrowax® HRC), Glycerintripalmitat oder die unter der Bezeichnung Syncrowax® HGLC bekannten Triglycerid-Gemische, mit der Vorgabe, dass der Schmelzpunkt der Wachskomponente bzw. des Gemisches bei 30 °C oder darüber liegt.

Als Wachskomponenten sind erfindungsgemäß insbesondere Mono- und Diglyceride bzw. Mischungen dieser Partialglyceride einsetzbar. Zu den erfindungsgemäß einsetzbaren Glyceridgemischen zählen die von der Cognis GmbH vermarkteten Produkte Novata AB und Novata B (Gemisch aus C₁₂-C₁₈-Mono-, Di- und Triglyceriden) sowie Cutina MD oder Cutina GMS (Glycerylstearat).

Weitere Wachskomponenten sind aus der Gruppe der C6-C22-Fettsäureester des Pentaerythrits, des Dipentaerythrits, des Tripentaerythrits oder eines beliebigen Gemisches dieser Ester, die einen Schmelzpunkt von wenigstens 30°C aufweisen. Ein bevorzugtes Pentaerythritsestergemisch besteht aus einem Anteil von 5 - 35 Gew.-% Monoester, 20 - 50 Gew.-% Diester und 25 - 50 Gel.-% Triester, und ggf. Tetraester. Besonders bevorzugt ist ein Gehalt an 10 - 25 Gew.-% Monoester, 25 - 40 Gew.-% Diester und 30 - 45 Gew.-% Triester, und ggf. Tetraester und ganz besonders bevorzugt 12 - 19 Gel.-% Monoester, 25 - 35 Gew.-% Diester und 30 - 40 Gew.-% Triester und 6 - 11 Gew.-% Tetraester. Zu dem erfindungsgemäß einsetzbarem Pentaerythritsestergemisch zählt das von der Cognis GmbH vermarktete Produkt ist Cutina^{®} PES.

Zu den erfindungsgemäß als Wachskomponente einsetzbaren Fettalkoholen zählen die C₁₂-C₅₀-Fettalkohole. Die Fettalkohole können aus natürlichen Fetten, Ölen und Wachsen gewonnen werden, wie beispielsweise Myristylalkohol, 1-Pentadecanol, Cetylalkohol, 1-Heptadecanol, Stearylalkohol, 1-Nonadecanol, Arachidylalkohol, 1-Heneicosanol, Behenylalkohol, Brassidylalkohol, Lignocerylalkohol, Cerylalkohol oder Myricylalkohol. Erfindungsgemäß bevorzugt sind gesättigte unverzweigte Fettalkohole. Aber auch ungesättigte, verzweigte oder unverzweigte Fettalkohole können erfindungsgemäß als Wachskomponente verwendet werden, solange sie den geforderten Schmelzpunkt aufweisen. Erfindungsgemäß einsetzbar sind auch Fettalkoholschnitte, wie sie bei der Reduktion natürlich vorkommender Fette und Öle wie z. B. Rinderialg, Erdnußöl, Rüböl, Baumwollsaatöl, Sojaöl, Sonnenblumenöl, Palmkernöl, Leinöl, Rizinusöl, Maisöl, Rapsöl, Sesamöl, Kakaobutter und Kokosfett anfallen. Es können aber auch synthetische Alkohole, z. B. die linearen, geradzahligen Fettalkohole der Ziegler-Synthese (Alfole) oder die teilweise verzweigten Alkohole aus der Oxosynthese (Dobanole) verwendet werden. Erfindungsgemäß besonders bevorzugt geeignet sind C₁₄-C₂₂-Fettalkohole, die beispielsweise von der Cognis GmbH unter der Bezeichnung Lanette 18 (C₁₈-Alkohol), Lanette 16 (C₁₆-Alkohol), Lanette 14 (C₁₄-Alkohol), Lanette O (C₁₆/C₁₈-Alkohol) und Lanette 22 (C₁₈/C₂₂-Alkohol) vermarktet werden. Fettalkohole verleihen den Zubereitungen ein trockeneres Hautgefühl als Triglyceride und sind daher gegenüber letzteren bevorzugt.

Als Wachskomponenten können auch C₁₄-C₄₀-Fettsäuren oder deren Gemische eingesetzt werden. Hierzu gehören beispielsweise Myristin-, Pentadecan-, Palmitin-, Margarin-, Stearin-, Nonadecan-, Arachin-, Behen-, Lignocerin-, Cerotin-, Melissin-, Eruca- und Elaeostearinsäure sowie substituierte Fettsäuren, wie z. B. 12-Hydroxystearinsäure, und die Amide oder Monoethanolamide der Fettsäuren, wobei diese Aufzählung beispielhaften und keinen beschränkenden Charakter hat.

Erfindungsgemäß verwendbar sind beispielsweise **natürliche pflanzliche Wachse**, wie Candelillawachs, Camaubawachs, Japanwachs, Espartograswachs, Korkwachs, Guarumawachs, Reiskeimölwachs, Zuckerrohrwachs, Ouricurywachs, Montanwachs, Sonnenblumenwachs, Fruchtwachse wie Orangenwachse, Zitronenwachse, Grapefruitwachs, Lorbeerwachs (=Bayberrywax) und **tierische Wachse**, wie z. B. Bienenwachs, Schellackwachs, Walrat, Wollwachs und Bürzelfett. Im Sinne der Erfindung kann es vorteilhaft sein, hydrierte oder gehärtete Wachse einzusetzen. Zu den erfindungsgemäß verwendbaren natürlichen Wachsen zählen auch die **Mineralwachse**, wie z. B. Ceresin und Ozokerit oder die petrochemischen Wachse, wie z. B. Petrolatum, Paraffinwachse und Mikrowachse. Als Wachskomponente sind auch **chemisch modifizierte Wachse**, insbesondere die Hartwachse, wie z. B. Montanesterwachse, Sasolwachse und hydrierte Jojobawachse einsetzbar. Zu den **synthetischen Wachsen**, die erfindungsgemäß einsetzbar sind, zählen beispielsweise wachsartige Polyalkylenwachse und Polyethylenglycolwachse. Pflanzliche Wachse sind erfindungsgemäß bevorzugt.

Die Wachskomponente kann ebenso gewählt werden aus der Gruppe der Wachsester aus gesättigten und/oder ungesättigten, verzweigten und/oder unverzweigten Alkancarbonsäuren und gesättigten und/oder ungesättigten, verzweigten und/oder unverzweigten Alkoholen, aus der Gruppe der Ester aus aromatischen Carbonsäuren, Dicarbonsäuren, Tricarbonsäuren bzw. Hydroxycarbonsäuren (z. B. 12-Hydroxystearinsäure) und gesättigten und/oder ungesättigten, verzweigten und/oder unverzweigten Alkoholen, sowie ferner aus der Gruppe der Lactide langkettiger Hydroxycarbonsäuren. Beispiel solcher Ester sind die C₁₆-C₄₀-Alkylstearate, C₂₀-C₄₀-Alkylstearate (z. B. Kesterwachs K82H), C₂₀-C₄₀-Dialkylester von Dimersäuren, C₁₈-C₃₈-Alkylhydroxystearoylstearate oder C₂₀-C₄₀-Alkylerucate. Ferner sind C₃₀-C₅₀-Alkylbienenwachs, Tristearylcitrat, Triisostearylcitrat, Stearylheptanoat, Stearyloctanoat, Trilaurylcitrat, Ethylenglycoldipalmitat, Ethylenglycoldistearat, Ethylenglykoldi(12-hydroxystearat), Stearylstearat, Palmitylstearat, Stearylbehenat, Cetylester, Cetearylbehenat und Behenylbehenat einsetzbar. Auch Fettsäurepartialglyceride, d. h. technische Mono- und/oder Diester des Glycerins mit Fettsäuren mit 12 bis 18 Kohlenstoffatomen wie etwa Glycerinmono/dilaurat, -palmitat, - myristat oderstearat kommen hierfür in Frage.

Als Wachse eignen sich weiterhin **Perlglanzwachse.** Als Perlglanzwachse, insbesondere für den Einsatz in tensidischen Formulierungen, kommen beispielsweise in Frage: Alkylenglycolester, speziell Ethylenglycoldistearat; Fettsäurealkanolamide, speziell Kokosfettsäurediethanolamid; Partialglyceride, speziell Stearinsäuremonoglycerid; Ester von mehrwertigen, gegebenenfalls hydroxy-substituierte Carbonsäuren mit Fettalkoholen mit 6 bis 22 Kohlenstoffatomen, speziell langkettige Ester der Weinsäure; Fettstoffe, wie beispielsweise Fettalkohole, Fettketone, Fettaldehyde, Fettether und Fettcarbonate, die in Summe mindestens 24 Kohlenstoffatome aufweisen, speziell Lauron und Distearylether; Fettsäuren wie Stearinsäure, Hydroxystearinsäure oder Behensäure, Ringöffnungsprodukte von Olefinepoxiden mit 12 bis 22 Kohlenstoffatomen mit Fettalkoholen mit 12 bis 22 Kohlenstoffatomen und/oder Polyolen mit 2 bis 15 Kohlenstoffatomen und 2 bis 10 Hydroxylgruppen sowie deren Mischungen.

Es kann erfindungsgemäß ein Wachs oder ein Gemisch aus mehreren Wachsen verwendet werden.

### Tenside

In einer Ausführungsform der Erfindung enthalten die erfindungsgemäßen Zubereitungen mindestens ein Tensid.

Tenside sind amphiphile Stoffe, die organische, unpolare Substanzen in Wasser lösen können. Sie sorgen, bedingt durch ihren spezifischen Molekülaufbau mit mindestens einem hydrophilen und einem hydrophoben Molekuelteil, für eine Herabsetzung der Oberflächenspannung des Wassers, die Benetzung der Haut, die Erleichterung der Schmutzentfemung und -lösung, ein leichtes Abspülen und - je nach Wunsch- für Schaumregulierung.

Als Tenside werden üblicherweise oberflächenaktive Substanzen verstanden, die einen HLB-Wert von größer 20 aufweisen.

Als oberflächenaktive Stoffe können anionische, nichtionische, kationische und/oder amphotere bzw. zwitterionische Tenside enthalten sein. In tensidhaltigen kosmetischen Zubereitungen ist vorzugsweise wenigstens ein anionisches Tensid enthalten.

Die erfindungsgemäßen Zubereitungen können die Tenside üblicherweise in einer Menge von 0 bis 40 Gew.-%., vorzugsweise 0,05 bis 30 Gew.-% insbesondere 0,05 bis 20 Gew.-%, vorzugsweise 0,1 bis 15 Gew.-% und insbesondere 0,1 bis 10 Gew.-%, bezogen auf das Gesamtgewicht der Zubereitung, enthalten.

Typische Beispiele für **nichtionische Tenside** sind Fettalkoholpolyglycolether, Alkylphenolpolyglycolether, Fettsäurepolyglycolester, Fettsäureamidpolyglycolether, Fettamin-polyglycolether, alkoxylierte Triglyceride, Mischether bzw. Mischformale, gegebenenfalls partiell oxidierte Alk(en)yloligoglykoside bzw. Glucoronsäurederivate, Feftsäure-N-alkylglucamide, Proteinhydrolysate (insbesondere pflanzliche Produkte auf Weizenbasis), Polyolfettsäureester, Zuckerester, Sorbitanester, Polysorbate und Aminoxide. Sofern die nichtionischen Tenside Polyglycoletherketten enthalten, können diese eine konventionelle, vorzugsweise jedoch eine eingeengte Homologenverteilung aufweisen.

Als **zwitterionische Tenside** werden solche oberflächenaktiven Verbindungen bezeichnet, die im Molekül mindestens eine quartäre Ammoniumgruppe und mindestens eine -COO⁽⁻⁾- oder -SO₃⁽⁻⁾-Gruppe tragen. Besonders geeignete zwitterionische Tenside sind die so genannten Betaine wie die N-Alkyl-N,N-dimethylammoniumglycinate, beispielsweise das Kokosalkyldimethylammoniumglycinat, N-Acyl-aminopropyl-N,N-dimethylammoniumglycinate, beispielsweise das Kokosacylaminopropyldimethylammoniumglycinat, und 2-Alkyl-3-carboxylmethyl-3-hydroxyethylimidazolin mit jeweils 8 bis 18 C-Atomen in der Alkyl- oder Acylgruppe sowie das Kokosacylaminoethylhydroxyethylcarboxymethylglycinat. Ein bevorzugtes zwitterionisches Tensid ist das unter der INCI-Bezeichnung Cocamidopropyl Betaine bekannte Fettsäureamid-Derivat.

Ebenfalls, insbesondere als Co-Tenside geeignet, sind **ampholytische Tenside**. Unter ampholytischen Tensiden werden solche oberflächenaktiven Verbindungen verstanden, die außer einer C₈-C₁₈-Alkyl- oder Acylgruppe im Molekül mindestens eine freie Aminogruppe und mindestens eine -COOH- oder - SO₃H-Gruppe enthalten und zur Ausbildung innerer Salze befähigt sind. Beispiele für geeignete ampholytische Tenside sind N-Alkylglycine, N-Alkylpropionsäuren, N-Alkylaminobuttersäuren, N-Alkyliminodipropionsäuren, N-Hydroxyethyl-N-alkylamidopropylglycine, N-Alkyltaurine, N- Alkylsarcosine, 2-Alkylaminopropionsäuren und Alkylaminoessigsäuren mit jeweils etwa 8 bis 18 C-Atomen in der Alkylgruppe. Besonders bevorzugte ampholytische Tenside sind das N-Kokosalkylaminopropionat, das Kokosacylaminoethylaminopropionat und das C₁₂₋₁₈-Acylsaroosin.

Typische Beispiele für **amphotere bzw. zwitterionische Tenside** sind Alkylbetaine, Alkylamidobetaine, Aminopropionate, Aminoglycinate, lmidazoliniumbetaine und Sulfobetaine. Bei den genannten Tensiden handelt es sich ausschließlich um bekannte Verbindungen. Hinsichtlich Struktur und Herstellung dieser Stoffe sei auf einschlägige Übersichtsarbeiten auf diesem Gebiet verwiesen. Typische Beispiele für besonders geeignete milde, d.h. besonders hautverträgliche Tenside sind Fettalkoholpolyglycolethersulfate, Monoglyceridsulfate, Mono- und/oder Dialkylsulfosuccinate, Fettsäureisethionate, Fettsäuresarcosinate, Fettsäuretauride, Fettsäureglutamate, α-Olefinsulfonate, Ethercarbonsäuren, Alkyloligoglucoside und/ oder deren Gemische mit Alkylollgoglucosidcarboxylaten, Fettsäureglucamide, Alkylamidobetaine, Amphoacetale und/oder Proteinfettsäurekondensate, letztere vorzugsweise auf Basis von Weizenproteinen bzw. deren Salzen.

**Anionische Tenside** sind gekennzeichnet durch eine wasserlöslich machende, anionische Gruppe wie z. B. eine Carboxylat-, Sulfat-, Sulfonat- oder Phosphat-Gruppe und einen lipophilen Rest. Hautverträgliche anionische Tenside sind dem Fachmann in großer Zahl aus einschlägigen Handbüchern bekannt und im Handel erhältlich. Es handelt sich dabei insbesondere um Alkylsulfate in Form ihrer Alkali-, Ammonium- oder Alkanolammoniumsalze, Alkylethersulfate, Alkylethercarboxylate, Acylisethionate, Acylsarkosinate, Acyltaurine mit linearen Alkyl- oder Acylgruppen mit 12 bis 18 C-Atomen sowie Sulfosuccinate und Acylglutamate in Form ihrer Alkali- oder Ammoniumsalze.

Typische Beispiele für **anionische Tenside** sind Seifen, Alkylbenzolsulfonate, Alkansulfonate, Olefinsulfonate, Alkylethersulfonate, Glycerinethersulfonate, α-Methylestersulfonate, Sulfofettsäuren, Alkylsulfate, Fettalkoholethersulfate, Glycerinethersulfate, Fettsäureethersulfate, Hydroxymischethersulfate, Monoglycerid(ether)sulfate, Fettsäureamid(ether)sulfate, Mono- und Dialkylsulfosuccinate, Mono- und Dialkylsulfosuccinamate, Sulfotriglyceride, Amidseifen, Ethercarbonsäuren und deren Salze, Fettsäureisethionate, Fettsäuresarcosinate, Fettsäuretauride, N-Acylaminosäuren, wie beispielsweise Acyllactylate, Acyltartrate, Acylglutamate und Acylaspartate, Alkyloligoglucosidsulfate, Proteinfettsäurekondensate (insbesondere pflanzliche Produkte auf Weizenbasis) und Alkyl(ether)phosphate. Sofern die anionischen Tenside Polyglycoletherketten enthalten, können diese eine konventionelle, vorzugsweise jedoch eine eingeengte Homologenverteilung aufweisen.

Als **kationische Tenside** sind insbesondere quartäre Ammoniumverbindungen verwendbar. Bevorzugt sind Ammoniumhalogenide, insbesondere Chloride und Bromide, wie Alkyltrimethylammoniumchloride, Dialkyldimethylammoniumchloride und Trialkylmethylammoniumchloride, z. B. Cetyltrimethylammoniumchlorid, Stearyltrimethylammoniumchlorid, Distearyldimethylammoniumchlorid, Lauryldimethylammoniumchlorid, Lauryldimethylbenzylammoniumchlorid und Tricetylmethylammoniumchlorid. Weiterhin können die sehr gut biologisch abbaubaren quatemären Esterverbindungen, wie beispielsweise die unter dem Warenzeichen Stepantex^{®} vertriebenen Dialfrylammoniummethosulfate und Methylhydroxyalkyldialkoyloxyalkylammoniummethosulfate und die entsprechenden Produkte der Dehyquart^{®}-Reihe, als kationische Tenside eingesetzt werden. Unter der Bezeichnung "Esterquats" werden im allgemeinen quaternierte Fettsäuretri-ethanolaminestersalze verstanden. Sie können den erfindungsgemäßen Zubereitungen einen besonderen Weichgriff verleihen. Es handelt sich dabei um bekannte Stoffe, die man nach den einschlägigen Methoden der organischen Chemie herstellt. Weitere erfindungsgemäß verwendbare kationische Tenside stellen die quatemisierten Proteinhydrolysate dar.

Neben den genannten Bestandteilen können andere, in kosmetischen Zubereitungen und insbesondere Antiperspirant/Deodorant-Formulierungen übliche Bestandteile in üblichen Anteilen enthalten sein. Hierzu gehören z.B. Konservierungsmittel, biogene Wirkstoffe, Verdickungsmittel, Überfettungsmittel, Stabilisatoren, Polymere, Antioxidantien, Filmbildner, Quellmittel, Insektenrepellentien, Hydrotrope, Solubilisatoren, Parfümöle, Farbstoffe, Pigmente, UV-Filter etc.

Die erfindungsgemäßen Antiperspirant/Deodorant-Zubereitungen enthalten die weiteren Bestandteile üblicherweise in Mengen von insgesamt < 25 Gew.%, insbesondere < 20 Gew.%, bezogen auf das Gesamtgewicht der Antiperspirant-/Desodorant-Zubereitung.

Als Konservierungsmittel eignen sich beispielsweise Phenoxyethanol, Formaldehydlösung, Parabene, Mischungen aus Phenoxyethanol und Ethylhexylgiycerin (wie sie beispielsweise unter dem Handelsnamen Euxyl PE 9010 erhältlich sind) oder Sorbinsäure sowie die unter der Bezeichnung Surfacine® bekannten Silberkomplexe und die in Anlage 6, Teil A und B der Kosmetikverordnung aufgeführten weiteren Stoffklassen.

In einer bevorzugten Ausführungsform der Erfindung wird das Konservierungsmittel ausgewählt aus der Gruppe bestehend aus Phenoxyethanol, Formaldehydlösung, Parabene, organische Säuren und Mischungen daraus, gegebenenfalls in Kombination mit Pentandiol und/oder Ethylhexylglycerin.

In einer Ausführungsform der Erfindung enthalten die erfindungsgemäßen Zubereitungen als weiteren Bestandteil mindestens einen biogenen Wirkstoff. Unter biogenen Wirkstoffen sind beispielsweise Tocopherol, Tocopherolacetat, Tocopherolpalmitat, Ascorbinsäure, (Desoxy)Ribonucleinsäure und deren Fragmentierungsprodukte, β-Glucane, Retinol, Bisabolol, Allantoin, Phytantriol, Panthenol, AHA-Säuren, Aminosäuren, Ceramide, Pseudoceramide, essentielle Öle, Pflanzenextrakte, wie z. B. Prunusextrakt, Bambaranussextrakt und Vitaminkomplexe zu verstehen.

In einer bevorzugten Ausführungsform der Erfindung enthalten die erfindungsgemäßen Zubereitungen als biogenen Wirkstoff mindestens eine Verbindung ausgewählt aus Vitaminen, Allantoin, Bisabolol und Pflanzenextrakten.

In einer bevorzugten Ausführungsform der Erfindung enthalten die erfindungsgemäßen Zubereitungen als biogenen Wirkstoff mindestens eine Verbindung ausgewählt aus Tocopherol, Tocopherolacetat, Tocopherolpalmitat, Ascorbinsäure, (Desoxy)Ribonucleinsäuren und deren Fragmentierungsprodukten, β-Glucanen, Retinol, Bisabolol, Allantoin, Phytantriol, Panthenol, AHA-Säuren, Aminosäuren, Ceramiden, Pseudoceramiden, essentiellen Ölen, Pflanzenextrakten, wie z. B. Aloe vera, Prunusextrakt, Bambaranussextrakt und Vitaminkomplexe und Mischungen daraus.

In einer Ausführungsform der Erfindung enthalten die erfindungsgemäßen Antiperspirant/Deodorant-Zubereitungen als weiteren Bestandteil mindestens ein Verdickungsmittel.

Als Verdickungsmittel eignen sich beispielsweise Aerosil-Typen (hydrophile Kieselsäuren), Polysaccharide, insbesondere Xanthan-Gum, Guar-Guar, Agar-Agar, Alginate und Tylosen, Carboxymethylcellulose und Hydroxyethylund Hydroxypropylcellulose, Polyvinylalkohol, Polyvinylpyrrolidon und Bentonite wie z. B. Bentone® Gel VS-5PC (Rheox).

Als Insekten-Repellentien kommen beispielsweise N,N-Diethyl-m-toluamid, 1 ,2-Pentandiol oder 3-(N-n-Butyl-N-acetyl-amino)-propionsäureethylester), welches unter der Bezeichnung Insect Repellent® 3535 von der Merck KGaA vertrieben wird, sowie Butylacetylaminopropionate in Frage.

Als Parfümöle seien genannt Gemische aus natürlichen und synthetischen Riechstoffen. Natürliche Riechstoffe sind Extrakte von Blüten, Stengeln und Blättern, Früchten, Fruchtschalen, Wurzeln, Hölzern, Kräutern und Gräsern, Nadeln und Zweigen, Harzen und Balsamen. Weiterhin kommen tierische Rohstoffe, wie beispielsweise Zibet und Castoreum sowie synthetische Riechstoffverbindungen vom Typ der Ester, Ether, Aldehyde, Ketone, Alkohole und Kohlenwasserstoffe in Frage.

Der Begriff **Pigment** umfasst Partikel jeder Form, die weiß oder gefärbt, organisch oder anorganisch sind, in den Zubereitungen nicht löslich sind, und dem Zweck dienen, die Zubereitung zu färben.
In einer bevorzugten Ausführungsform werden anorganische Pigmente verwendet, besonders bevorzugt sind Metalloxide.

Als **anorganische Pigmente** seien exemplarisch genannt: Titandioxid, optional oberflächenbeschichtet, Zirkonium oder Ceriumoxide und Zink-, Eisen- (Schwarz, Gelb oder Rot) und Chromoxide, Manganviolett, Ultramarine Blau, Chromhydrate und Eisen(III)blau, Metallpulver wie Aluminiumpulver oder Kupferpulver.
In einer bevorzugten Ausführungsform der Erfindung ist das Pigment ausgewählt aus den anorganischen Pigmenten, vorzugsweise aus den Metalloxiden. In einer bevorzugten Ausführungsform ist das Pigment ausgewählt aus der Gruppe bestehend aus Titandioxid, Zinkoxid, Eisenoxid und Mischungen daraus.
Die Pigmente können sowohl einzeln als auch in Mischungen vorliegen.
Bevorzugt im Sinne der vorliegenden Erfindung sind Pigmentmischungen aus Weiß-Pigmenten (z. B. Kaolin, Titandioxid oder Zinkoxid) und anorganischen Farbpigmenten (z. B. Eisenoxid Pigmente, Chromoxide), wobei die Pigmente beschichtet ("gecoatet") oder unbeschichtet vorliegen können. Unter den Farbpigmenten sind Eisenoxide besonders bevorzugt.

Vorteilhaft im Sinne der vorliegenden Erfindung können das oder die Pigmente auch aus der Gruppe der Effektpigmente gewählt werden, welche der kosmetischen Zubereitung neben der reinen Farbe eine zusätzliche Eigenschaft - wie z. B. eine Winkelabhängigkeit der Farbe (changieren, Flop), Glanz (nicht Oberflächenglanz) oder Textur - verleihen. Solche Effektpigmente werden erfindungsgemäss vorteilhaft zusätzlich zu einem oder mehreren Weiss- und/oder Farbpigmenten eingesetzt.

Die bedeutendste Gruppe der Effektpigmente stellen die Glanzpigmente dar, zu denen nach DIN 55944: 2003-11 die Metalleffektpigmente und die Perlglanzpigmente gehören. Einige spezielle Effektpigmente lassen sich diesen beiden Gruppen nicht zuordnen, z. B. plättchenfoermiges Graphit, pläftchenförmiges Eisenoxid und mikronisiertes Titandioxid, wobei mikronisiertes Titandioxid keinen Glanzeffekt, sondern einen winkelabhängigen Lichtstreueffekt erzeugt. Bei den Glanzpigmenten nach DIN 55943: 2001-10 handelt es sich vorwiegend um plättchenfoermige Effektpigmente. Parallel orientiert zeigen Glanzpigmente einen charakteristischen Glanz. Die optische Wirkung von Glanzpigmenten beruht auf der gerichteten Reflexion an metallischen Teilchen (Metalleffektpigmente), an transparenten Teilchen mit hoher Brechzahl (Perlglanzpigmente) oder auf dem Phaenomen der Interferenz (Interferenzpigmente) (DIN 55944: 2003-11).

Beispiele für erfindungsgemäss bevorzugte handelsübliche Effektpigmente sind: Timiron and #174; von Merck, Iriodin and #174; von Merck (Perl- und Farbglanzpigmente für dekorative technische Anwendungen), Xirallic and #174; von Merck (farbintensive Kristalleffektpigmente).

Ferner können die erfindungsgemäßen Zubereitungen vorteilhaft auch **organische Farbpigmente** enthalten, d. h. organische Farbstoffe, welche in der Zubereitung praktisch unlöslich sind. Nach DIN 55944: 1990-04 können organische Pigmente nach chemischen Gesichtspunkten in Azopigmente und polycyclische Pigmente sowie nach farblichen Gesichtspunkten in Bunt- oder Schwarzpigmente eingeteilt werden. Organische Weißpigmente sind ohne praktische Bedeutung.
Die Pigmente können vorteilhaft im Sinne der vorliegenden Erfindung auch in Form kommerziell erhältlicher öliger oder wässriger Vordispersionen zur Anwendung kommen.
Die erfindungsgemäßen Zubereitungen können bspw. 0,1 bis 40 Gel.-% Pigmente - bezogen auf das Gesamtgewicht der kosmetischen und/oder pharmazeutischen Zubereitung enthalten.

Es ist ferner möglich, dass die erfindungsgemäße Zubereitung einen oder mehrere **Farbstoffe** enthält.

Die Farbstoffe können sowohl synthetischen als auch natürlichen Ursprungs sein.
Eine Liste von geeigneten Farbstoffen findet sich in EP 1 371 359 A2, S.8, Z. 25-57, S.9 und S.10 sowie S.11, Z.1 bis 54, auf welche hiermit explizit Bezug genommen wird.

Die erfindungsgemäßen Zubereitungen enthalten üblicherweise 0,01 bis 5, vorzugsweise 0,1 bis 1,0 Gew.-% Farbstoffe- bezogen auf das Gesamtgewicht der kosmetischen und/oder pharmazeutischen Zubereitung. Die erfindungsgemäßen Zubereitungen können beispielsweise eine Gesamtmenge an Farbstoffen und Pigmenten im Bereich von 0,01 bis 30 Gel.-%, insbesondere 0,1 bis 15 Gew.-%, vorzugsweise 1 bis 10 Gew.-%, bezogen auf das Gesamtgewicht der kosmetischen und/oder pharmazeutischen Zubereitung enthalten.

Als Farbstoffe und Pigmente eignen sich insbesondere die gemäß Annex IV der Kommissions Direktive zugelassenen Farbstoffe und Pigmente (in der Fassung: Commission Directive 2007/22/EC of 17 April 2007 amending Council Directive 76/768/EEC, conceming cosmetic products, for the purposes of adapting Annexes IV and VI thereto to technical progress), auf die hiermit explizit Bezug genommen wird.

Erfindungsgemäß sind als **UV-Lichtschutzfilter** bei Raumtemperatur flüssige oder kristalline organische Substanzen (Lichtschutzfilter) geeignet, die in der Lage sind, ultraviolette Strahlen zu absorbieren und die aufgenommene Energie in Form längerwelliger Strahlung, z.B. Wärme wieder abzugeben. UV-Filter können öllöslich oder wasserlöslich sein. Als typische öllösliche UV-B-Filter bzw. Breitspektrum-UV A/B-Filter sind z.B. zu nennen:
➢ 3-Benzylidencampher bzw. 3-Benzylidennorcampher (Mexoryl SDS 20) und dessen Derivate, z.B. 3-(4-Methylbenzyliden)campher wie in der EP 0693471 B1 beschrieben
➢ 3-(4'-Trimethylammonium) benzyliden- boman-2-on-methylsulfat (Mexoryl SO)
➢ 3,3'-(1,4-Pheny(endimethin)-bis (7,7- dimethyl-2-oxobicyclo-[2.2.1] heptan-1-methansulfonsäure) and salts (Mexoryl SX)
➢ 3-(4'-Sulfo)-benzyliden-bornan-2-on and salts (Mexoryl SL)
➢ Polymer von N-{(2und 4)- [2-oxoborn-3-yliden)methyl}benzyl]acrylamid (Mexoryl SW)
➢ 2-(2H-Benzotriazol-2-yl)-4-methyl-6-(2-methyl-3-(1,3,3,3-tetramethyl-1-(trimethylsilyloxy) disiloxanyl)propyl) phenol (Mexoryl SL)
➢ 4-Aminobenzoesäurederivate, vorzugsweise 4-(Dimethylamino)benzoesäure-2-ethyl-hexylester, 4-(Dimethylamino)benzoesäure-2-octylester und 4-(Dimethylamino)benzoe-säureamylester;
➢ Ester der Zimtsäure, vorzugsweise 4-Methoxyzimtsäure-2-ethylhexylester, 4-Methoxy-zimtsäurepropylester, 4-Methoxyzimtsäureisoamylester, 2-Cyano-3,3-phenylzimtsäure-2-ethylhexylester (Octocrylene);
➢ Ester der Salicylsäure, vorzugsweise Salicylsäure-2-ethylhexylester, Salicylsäure-4-iso-propylbenzylester, Salicylsäurehomomenthylester;
➢ Derivate des Benzophenons, vorzugsweise 2-Hydroxy-4-methoxybenzophenon, 2-Hydroxy-4-methoxy-4'-methylbenzophenon, 2,2'-Dihydroxy-4-methoxybenzophenon;
➢ Ester der Benzalmalonsäure, vorzugsweise 4-Methoxybenzmalonsäuredl-2-ethylhexyl-ester,
➢ Triazinderivate, wie z.B. 2,4,6-Trianilino-(p-carbo-2'-ethyl-1'-hexyloxy)-1,3,5-triazin und 2,4,6-Tris[p-(2-ethylhexyl-oxycar-bonyl) anilino]-1,3,5-triazin (Uvinul T 150) wie in der EP 0818450 A1 beschrieben oder 4,4'-[(6-[4-((1,1-Dimethylethyl)amino-carbonyl) phenyl-amino]-1,3,5-triazin-2,4-diyl)diimino] bis(benzoesäure-2- ethylhexylester) (Uvasorb® HEB);
➢ 2,2(-Methylen-bis(6-(2H-benzotriazol-2-yl)-4- (1,1,3,3-tetramethyl-butyl)phenol) (Tinosorb M);
➢ 2,4-Bis[4-(2-ethylhexyloxy)-2- hydroxyphenyl]-6-(4- methoxyphenyl)-1,3,5- triazin (Tinosorb S);
➢ Propan-1,3-dione, wie z.B. 1-(4-tert.Butylphenyl)-3-(4'methoxyphenyl)propan-1,3-dion;
➢ Ketotricyclo(5.2.1.0)decan-Derivate, wie in der EP 0694521 B1 beschrieben;
➢ Dimethicodiethylbenzalmalonate (Parsol SLX).

Als wasserlösliche UV - Filter kommen in Frage:
➢ 2-Phenylbenzimidazol-5-sulfonsäure und deren Alkali-, Erdalkali-, Ammonium-, Alkylammonium-, Alkanolammonium- und Glucammoniumsalze;
➢ 2,2(-(1,4-Pheny)en)bis(1H-benzimidazol- 4,6-disulfon-säure, Mononatriumsalz) (Neo Heliopan AP)
➢ Sulfonsäurederivate von Benzophenonen, vorzugsweise 2-Hydroxy-4-methoxybenzo-phenon-5-sulfonsäure und ihre Salze;
➢ Sulfonsäurederivate des 3-Benzylidencamphers, wie z.B. 4-(2-Oxo-3-bomylidenmethyl)-benzolsulfonsäure und 2-Methyl-5-(2-oxo-3-bomyliden)sulfonsäure und deren Salze.

Als typische UV-A-Filter kommen insbesondere Derivate des Benzoylmethans in Frage, wie beispielsweise 1-(4'-tert.Butylphenyl)-3-(4'-methoxyphenyl)propan-1,3-dion, 4-tert.-Butyl-4'-methoxydibenzoylmethan (Parsol® 1789), 1-Phenyl-3-(4'-isopropylphenyl)-propan-1,3-dion sowie Enaminverbindungen, wie beschrieben in der DE 19712033 A1 (BASF) sowie Benzoic Acid, 2-[4-(Diethylamino)-2-Hydroxybenzoyl]-, Hexyl Ester (Uvinul® A plus).

Die UV-A und UV-B-Filter können selbstverständlich auch in Mischungen eingesetzt werden. Besonders günstige Kombinationen bestehen aus den Derivaten des Benzoylmethans, z.B. 4-tert.-Butyl-4'-methoxydibenzoylmethan (Parsol® 1789) und 2-Cyano-3,3-phenylzimtsäure-2-ethyl-hexylester (Octocrylene) in Kombination mit Estern der Zimtsäure, vorzugsweise 4-Methoxyzimtsäure-2-ethylhexylester und/oder 4-Methoxyzimtsäurepropylester und/oder 4-Methoxyzimtsäureisoamylester. Vorteilhaft werden derartige Kombinationen mit wasserlöslichen Filtern wie z.B. 2-Phenylbenzimidazol-5-sulfonsäure und deren Alkall-, Erdalkali-, Ammonium-, Alkylammonium-, Alkanolammonium- und Glucammoniumsalze kombiniert.

Als UV-Lichtschutrfilter eignen sich insbesondere die gemäß Annex VII der Kommissions Direktive (in der Fassung **Commission Directive 2005/9/EC of 28 January 2005 amending Council Directive 76/768/EEC,** conceming cosmetic products, for the purposes of adapting Annexes VII thereof to technical progress) zugelassen Stoffe, auf die hier explizit Bezug genommen wird.

Neben den genannten löslichen Stoffen kommen für diesen Zweck auch unlösliche Lichtschutzpigmente, nämlich feindisperse Metalloxide bzw. Salze in Frage. Beispiele für geeignete Metalloxide sind insbesondere Zinkoxid und Titandioxid und daneben Oxide des Eisens, Zirkoniums, Siliciums, Mangans, Aluminiums und Cers sowie deren Gemische. Als Salze können Silicate (Talk), Bariumsulfat oder Zinkstearat eingesetzt werden. Die Oxide und Salze werden in Form der Pigmente für hautpflegende und hautschützende Emulsionen und auch für die dekorative Kosmetik verwendet. Die Partikel sollten einen mittleren Durchmesser von weniger als 100 nm, vorzugsweise zwischen 5 und 50 nm und insbesondere zwischen 15 und 30 nm aufweisen. Sie können eine sphärische Form aufweisen, es können jedoch auch solche Partikel zum Einsatz kommen, die eine ellipsoide oder in sonstiger Weise von der sphärischen Gestalt abweichende Form besitzen. Die Pigmente können auch oberflächenbehandelt, d.h. hydrophilisiert oder hydrophobiert vorliegen. Typische Beispiele sind gecoatete Titandioxide, wie z.B. Titandioxid T 805 (Degussa) oder Eusolex® T, Eusolex® T-2000, Eusolex® T-Aqua, Eusolex® AVO, Eusolex® T-ECO, Eusolex® T-OLEO und Eusolex® T-S (Merck). Typische Beispiele für sind Zinkoxide, wie z.B. Zinc Oxide neutral, Zinc Oxide NDM (Symrise) oder Z-Cote® (BASF) oder SUNZnO-AS und SUNZnO-NAS (Sunjun Chemical Co. Ltd.). Als hydrophobe Coatingmittel kommen dabei vor allem Silicone und dabei speziell Trialkoxyoctylsilane oder Simethicone in Frage. In Sonnenschutzmitteln werden bevorzugt sogenannte Mikro- oder Nanopigmente eingesetzt. Vorzugsweise wird mikronisiertes Zinkoxid verwendet. Weitere geeignete UV-Lichtschutzfilter sind der Übersicht von P. Finkel in SÖFW-Journal 122, 8/1996, S. 543-548 sowie Parf. Kosm. 80. Jahrgang, Nr. 3/1999, S. 10 bis 16 zu entnehmen.

Neben den beiden vorgenannten Gruppen primärer Lichtschutzstoffe können auch sekundäre Lichtschutzmittel vom Typ der **Antioxidantien** eingesetzt werden, die die photochemische Reaktionskette unterbrechen, welche ausgelöst wird, wenn UV-Strahlung in die Haut eindringt. Typische Beispiele hierfür sind Aminosäuren (z.B. Glycin, Histidin, Tyrosin, Tryptophan) und deren Derivate, Imidazole (z.B. Urocaninsäure) und deren Derivate, Peptide wie D,L-Carnosin, D-Carnosin, L-Carnosin und deren Derivate (z.B. Anserin), Carotinoide, Carotine (z.B. -Carotin, -Carotin, Lycopin) und deren Derivate, Chlorogensäure und deren Derivate, Liponsäure und deren Derivate (z.B. Dihydroliponsäure), Auro-thioglucose, Propylthiouracil und andere Thiole (z.B. Thloredoxin, Glutathion, Cystein, Cystin, Cysta-min und deren Glycosyl-, N-Acetyl-, Methyl-, Ethyl-, Propyl-, Amyl-, Butyl- und Lauryl-, Palmitoyl-, Oleyl-, -Linoleyl-, Cholesteryl- und Glycerylester) sowie deren Salze, Dilaurylthiodipropionat, Distearylthiodipropionat, Thiodipropionsäure und deren Derivate (Ester, Ether, Peptide, Lipide, Nukleotide, Nukleo-side und Salze) sowie Sulfoximinverbindungen (z.B. Buthioninsulfoximine, Homocysteinsulfoximin, Butioninsulfone, Penta-, Hexa-, Heptathioninsulfoximin) in sehr geringen verträglichen Dosierungen (z.B. pmol bis mol/kg), ferner (Metall)-Chelatoren (z.B. α-Hydroxyfettsäuren, Palmitinsäure, Phytinsäure, Lactoferrin), α-Hydroxysäuren (z.B. Citronensäure, Milchsäure, Äpfelsäure), Huminsäure, Gallensäure, Gallenextrakte, Billrubin, Biliverdin, EDTA, EGTA und deren Derivate, ungesättigte Fettsäuren und deren Derivate (z.B. gamma-Linolensäure, Linolsäure, Ölsäure), Folsäure und deren Derivate, Ubichinon und Ubichinol und deren Derivate, Vitamin C und Derivate (z.B. Ascorbylpalmitat, Mg-Ascor-bylphosphat, Ascorbylacetat), Tocopherole und Derivate (z.B. Vitamin-E-acetat), Vitamin A und Derivate (Vitamin-A-palmitat) sowie Koniferylbenzoat des Benzoeharzes, Rutinsäure und deren Derivate, α-Glycosylrutin, Ferulasäure, Furfurylidenglucitol, Camosin, Butylhydroxytoluol, Butylhydroxyanisol, Nordihydroguajakharzsäure, Nordihydroguajaretsäure, Trihydroxybutyrophenon, Harnsäure und deren Derivate, Mannose und deren Derivate, Superoxid-Dismutase, Zink und dessen Derivate (z.B. ZnO, ZnSO4) Selen und dessen Derivate (z.B. Selen-Methionin), Stilbene und deren Derivate (z.B. Stilbenoxid, trans-Stilbenoxid) und die erfindungsgemäß geeigneten Derivate (Salze, Ester, Ether, Zucker, Nukleotide, Nukleoside, Peptide und Lipide) dieser genannten Wirkstoffe.

Als Stabilisatoren können Metallsalze von Fettsäuren, wie z. B. Magnesium-, Aluminium- und/oder Zinkstearat bzw. -ricinoleat eingesetzt werden.

Zur Verbesserung des Fließverhaltens können ferner Hydrotrope, wie beispielsweise Ethanol, Isopropylalkohol, oder Polyole eingesetzt werden. Polyole, die hier in Betracht kommen, besitzen vorzugsweise 2 bis 15 Kohlenstoffatome und mindestens zwei Hydroxylgruppen. Die Polyole können noch weitere funktionelle Gruppen, insbesondere Aminogruppen, enthalten bzw. mit Stickstoff modifiziert sein.

Die weiteren Bestandteile der Zubereitungen (wie z.B. Konservierungsmittel, kosmetische Wirkstoffe, UV-Filter etc.) werden je nach ihrer Löslichkeit entweder über die Wasserphase oder über die lipophile Phase zugegeben.

### Wässriger Anteil:

Die erfindungsgemäßen Zubereitungen können üblicherweise 40 bis 85, bevorzugter 50 bis 75 Gew.-%, wässrigen Anteil, bezogen auf das Gesamtgewicht der Zubereitung, enthalten.

Die erfindungsgemäßen Zubereitungen liegen im Allgemeinen als Emulsionen vor, in der Regel in Form von Gelen oder Gelcremes (nicht transparente Gele). Geeignete Formen der erfindungsgemäßen Zubereitungen sind z.B. Antitranspirant-/Deosprays, Antittanspirant-/Deolotionen, Antitranspirant-/Deoroll-ons, Antitranspirant-/Deogele, Antitranspirant-/Deocremes oder Antitranspirant-/Deostifte.

### Beispiele

Die folgenden Beispiele sollen die vorliegende Erfindung näher erläutern.

### Herstellung der Antiperspirant-/Desodorant-Zubereitungen:

Die Quellung in der Wasserphase mit Polyquaternium 37 fand bei Raumtemperatur statt. Unterdessen wurde die lipophile Phase - abhängig von der Zusammensetzung - auf 60°C erwärmt und anschließend unter Rühren zu dem Polyquaternium 37 Gel hinzugegeben. Der Antiperspirant-/Desodorant-Wirkstoff wurde in das System bei einer Temperatur kleiner 45°C gegeben. Die Formulierung wurde nicht konserviert und der pH Wert lag zwischen 3.00 und 4.50.

### Bewertung der AP/Deo - Formulierungen:

Die Formulierungen wurden für 4 Wochen bei Raumtemperatur (RT) und 40°C gelagert.
Die Beurteilung der Formulierungen wurde wie folgt durchgeführt:
1 = stabil
2 = phasenstabil, aber das makroskopische Bild ist inhomogen (keine Öl- oder Wasserabscheidung)
3 = geringe Wasser- oder Ölabscheidung
4 = instabil (deutlich sichtbare Wasser bzw. Ölabscheidung)

### Sensorische Beurteilung der EO freien AP/Deo - Formulierungen:

Die jeweilige Formulierung wurde in einer definierten Menge auf den Unterarm aufgetragen und gleichmäßig bis zum ersten Einziehen verrieben. Bis zum vollständigen Einziehen wurde ca. 3 - 5 Minuten gewartet und anschließend das Hautgefühl bestimmt. Ein Panel bestehend aus 12 Experten führte die sensorische Bewertung durch. Abgefragt wurden die folgenden 6 Kriterien bezogen auf das Endgefühl auf der Haut:

| | |
|---|---|
| Verteilbarkeit | (1 = sehr gut; 7 = sehr schlecht) |
| Klebrigkeit | (1 = sehr gering; 7 = sehr stark) |
| wachsig | (1 = sehr gering; 7 = stumpf) |
| Glätte | (1 = hohes Glättegefühl; 7 = stumpf) |
| Weichheit | (1 = hohes Weichheitsgefühl; 7 = rau) |
| Pflege | (1 = hohes Pflegegefühl; 7 = ungepflegt) |

**Tab.1: Zusammensetzung und Bewertung erfindungsgemäßer AP/Deo-Zubereitungen**

| **INCI** | **Beispiel 1** | **Beispiel 2** | **Beispiel 3** | **Beispiel 4** | **Beispiel 5** |
|---|---|---|---|---|---|
| Dest Wasser | 55,00 % | 58,50 % | 58,50 % | 59,50 % | 59,00 % |
| Aluminiumchlorhydrat (50 %) | 30,00 % | 30,00 % | 30,00 % | 30,00 % | 30,00 % |
| Pentaerthrityldistearat | 3,00 % | 3,00 % | - | - | 3.50% |
| Cetylpalmitat | - | - | 3,00 % | - | - |
| Butyrospermum -Parkii (Shea Butter) | | - | - | 3,00 % | - |
| Octyldodecanol | 7,00 % | 4,00 % | 4,00 % | 4,00% | 4,00 % |
| Polyquaternium 37 | 5,00 % | 4,50 % | 4,50 % | 3,50 % | 3,50 % |

| **Bewertung der Stabilität** | | | | | |
|---|---|---|---|---|---|
| 1 Woche/RT | 1 | 1 | 1 | 1 | 1 |
| 1 Woche/40°C | 1 | 1 | 1 | 1 | 1 |
| 2 Wochen/RT | 1 | 1 | 1 | 1 | 1 |
| 2 Wochen/40°C | 1 | 1 | 1 | 1 | 1 |
| 4 Wochen/RT | 1 | 1 | 1 | 1 | 1 |
| 4 Wochen/40°C | 1 | 1 | 1 | 1 | 1 |

| **Bewertung der Sensorik** | | | | | |
|---|---|---|---|---|---|
| Verteilbarkeit | 3 | 2 | 5 | 3 | 3 |
| Klebrigkeit | 2 | 3 | 3 | 1 | 2 |
| wachsig | 1 | 2 | 5 | 1 | 1 |
| Glätte | 3 | 3 | 2 | 1 | 1 |
| Weichheit | 3 | 2 | 3 | 2 | 3 |
| Pflege | 2 | 2 | 2 | 3 | 3 |

Die erfindungsgemäßen Beispiele 1 bis 5 zeigen eine sehr gute Stabilität sowie vorteilhafte sensorische Eigenschaften (geringe Klebrigkeit, hohe Weichheit etc.).

**Tab. 2: Zusammensetzung und Bewertung einer AP/Deo-Zubereitungen gemäß Stand der Technik und von erfindungsgemäßen Formulierungen mit Polyquaternium 37**

| **INCI** | **Vergleichsbeispiel 1** | **Beispiel 6** | **Beispiel 7** |
|---|---|---|---|
| Dest. Wasser | 69,00% | 68,50 % | 72,00 |
| Aluminiumchlorhydrat (50 %) | 25,00 % | 25,00 % | 25,00 |
| Cetearylalcohol | 1,00 % | 1,00 % | - |
| Natriumstearoyiglutamat | 0,65 % | - | - |
| Propylheptylcaprylat | 1,00 % | 1,00 % | - |
| Dicaprylylcarbonat | 1,00 % | 1,00 % | - |
| Dimethicon | 0,50 % | 0,50 % | - |
| Hydroxyethylcellulose | 0,50 % | - | - |
| Glycerylstearatcitrat | 1,35 % | - | - |
| Polyquaternium 37 | - | 3,00 % | 3,00 % |

| **Bewertung der Stabilität** | | | |
|---|---|---|---|
| 1 Woche/RT | 1 | 1 | 1 |
| 1 Woche/40°C | 1 | 1 | 1 |
| 2 Wochen/RT | 1 | 1 | 1 |
| 2 Wochen/40°C | 4 | 1 | 1 |
| 4 Wochen/RT | 5 | 1 | 1 |
| 4 Wochen/40°C | 5 | 1 | 1 |

| **Bewertung der Sensorik** | | | |
|---|---|---|---|
| Verteilbarkeit | 4 | 2 | 3 |
| Klebrigkeit | 6 | 3 | 4 |
| wachsig | 5 | 2 | 1 |
| Glätte | 5 | 2 | 2 |
| Weichheit | 5 | 3 | 4 |
| Pflege | 5 | 2 | 3 |

Als Vergleichsbeispiel wurde eine Antiperspirant-/Desodorant-Zubereitung gewählt, welche kein Polyquatemium 37, aber Ethylenoxid-freien Emulgator enthält (Natriumstearoylglutamat und Gylcerylstearatcitrat). Es zeigte sich, dass die erfindungsgemäßen, Polyquaternium 37 enthaltenden Antiperspirant-/Desodorant-Zubereitungen der Beispiele 1 bis 5 ohne einen Emulgator nicht nur stabiler als die Vergleichsrezeptur waren, sondern gleichzeitig eine verbesserte Sensorik aufwiesen. Dabei blieb die Antiperspirant-/Desodorant-Wirkung erhalten, und die ökologischen Bedenken hinsichtlich Ethylenoxid konnten vermieden werden.

Die erfindungsgemäßen Beispiele 6 und 7 zeigten gegenüber dem Stand der Technik (Vergl.-Bsp. 1) ebenfalls eine verbesserte Stabilität, sowie eine verringerte Klebrigkeit und ein hohes Pflegegefühl.

Weitere erfindungsgemäße Rezepturbeispiele (R1 bis R11) sind nachfolgend aufgeführt:

| Komponente C - Creme, L - Lotion, F - Fluid | R1 | R2 | R3 | R4 | R5 | R6 | R7 | R8 | R9 | R10 | R11 |
|---|---|---|---|---|---|---|---|---|---|---|---|
| | C | C | C | L | C | L | L | C | L | C | C |
| Cutina^{®} PES INCl: pentaerythrityl Distearate Hersteller: Cognis GmbH | | 3 | 3 | 2 | 2 | 3 | 2 | | 2 | | 3 |
| Cutina^{®} MD INCl: Glyceryl Stearate Hersteller: Cognis GmbH | 2 | | | 2 | 3 | 1 | 1 | 3 | 2 | 3 | 1 |
| Lanette^{®} 14 INCI: Myristyl Alcohol Hersteller: Cognis GmbH | | | 2 | | | | | 2 | | 2,5 | 1 |
| Lanette^{®} 0 INCl: Cetearyl Alcohol Hersteller: Cognis GmbH | 1 | 2 | | 1 | 2 | 1 | 2,5 | | 1,5 | | |
| Novata^{®} AB INCl: Cocoglycerides Hersteller: Cognis GmbH | | 1 | 3 | | | | 2 | | | | 2,5 |
| Cetiol MM^{®} MM INCl: Myristyl Myristate Hersteller: Cognis GmbH | 3 | | | 2 | 2,5 | 3 | | 1 | 4 | | |
| Cutina^{®} CP INCI: Cetyl Palmitate Hersteller Cognis GmbH | | 3 | | | 1,5 | | 2 | 1 | | | 3 |
| Cosmedia^{®} DC INCl: Hydrogenated Dimer Dilinoleyl / Dimethylcarbonate Copolymer Hersteller: Cognis GmbH | | | 2 | | | | | | | 2,5 | |
| Cetiol^{®} SB 45 INCl: Shea Butter Butyrospermum Parkii Hersteller: Cognis GmbH | 1 | | | | | 1 | | | 1 | | |
| Myritol^{®} 318 INCl: Caprytic/Capric Triglyceride Hersteller: Cognis GmbH | | | 3 | | | 4 | | 5 | | | 2 |
| Myritol^{®} PC INCl: Propylene Glycol Dicaprylate/ Dicaprate Hersteller: Cognis GmbH | | | | 3 | | | 2 | | | 2 | |
| Myrital^{®} 331 INCl: Cocoglycerides Hersteller: Cognis GmbH | 4 | 3 | | | | | | 3 | | | |
| Finsolv^{®} TN INCl: C 12/15 Alkyl Benzoate Hersteller: Findex (Nordmann/Rassmann) | | | | | 3 | 4 | | | 2 | | |
| Cetiol^{®} CC INCl: Dicaprylyl Carbonate Hersteller: Cognis GmbH | 2 | | | | 3 | | | 4 | | | |
| Cetiol^{®} OE INCI: Dicaprylyl Ether Hersteller: Cognis GmbH | 3 | 4 | | | | 3 | | | 2 | | 5 |
| Dow Coming DC^{®} 245 INCl: Cyclopentasiloxane Hersteller: Dow Coming | | | | 1 | | | | | | | |
| Dow Coming^{®} 2502 INCl: Cetyl Dimethicone Hersteller: Dow Coming | | | | | 1 | | | | | | 1 |
| Prisorine^{®} 3758 INCl: Hydrogenated Polyisobutene Hersteller: Uniqema | | | 2 | | | | 4 | | | 2 | |
| Silikonöl Wacker AK^{®} 350 INCl:Dimethicone Hersteller: Wacker | | 0,5 | | | 1 | | | 0,5 | | | |
| Cetiol^{®} 868 INCl: Ethylhexyl Stearate Hersteller: Cognis GmbH | 3 | | | | 2 | | | 5 | | | |
| Cetiol^{®} J 600 INCl: Oleyl Erucate Hersteller: Cognis GmbH | | | | 4 | | 2 | | | 3 | 3 | 2 |
| Ceraphyl^{®} 45 INCl: Diethylhexyl Malate Hersteller: International Specialty Products | | | | | | 3 | | | | | |
| Mineralöl Hersteller: Exxon Mobil Lubricants and Petroleum Specialties oder Universal Preserv-A-Chem, Inc. oder Sonnebom LLC Refined Products etc. | | 3 | | 2 | | | | | | 3 | |
| Cetiol^{®} Sensoft INCl: Propylheptyl Caprylate Hersteller: Cognis GmbH | 2 | | | | 3 | 2 | | | 2 | | |
| Cetioi^{®} SN INCl: Cetearyl Isononanoate Hersteller: Cognis GmbH | | | 4 | | | | | 3 | | | |
| Cetiol^{®} B INCI: Dibutyl Adipate Hersteller: Cognis GmbH | | 3 | | | | | 2 | | | 3 | |
| Eutanol^{®} G INCl: Octyldodecanol Hersteller: Cognis GmbH | | | | | | 5 | 4 | | 3 | | |
| Cetiol^{®} PGL INCl: Hexyldecanol, Hexyldecyl Laurate Hersteller: Cognis GmbH | | | | 3 | | 1 | | | | | 4 |
| Dow Coming 200 - 2 cst INCl: Dimethicone Hersteller: Dow Coming | | | 1 | | | | | 2 | | 3 | |
| SFE^{®} 839 INCl: Cyclopentasiloxane and Dimethicone/Vinyl Dimethicone Crosspolymer Hersteller: GE Silicones | | | | | 3 | | | | 2 | | |
| Mandelöl INCl: Prunus Amygdalus Dulcis (Sweet Almond) Oil Hersteller: Croda Inc. oder Cosmetochem International Ltd. etc. | | 2 | | | 2,5 | | | 3 | | 2 | |
| Talkum Hersteller: Whittaker, Clark & Daniels, Inc., Rio Tinto Minerals (formerly Luzenac) oder Ultra Chemical, Inc., etc. | | | 0,5 | | | | | | 1 | | |
| Dry^{®}Fio Plus INCl: Aluminium Starch Octenylsuccinate Hersteller: National Starch | 1 | | | | | 0,5 | | | | | |
| Insect Repellent^{®} 3535 INCl: Ethyl Butylacetylaminopropionate Hersteller: EMD Chemicals Inc | | | 2 | | | | | | 1 | | 2 |
| N,N-Diethyl-m-toluamid Hersteller: Orient Stars LLC, Lipo Chemicals, Inc. oder Induchem AG | | 2 | | | | | | 2 | | | |
| Locron^{®} L INCl: Aluminium Chlorhydrate Hersteller: Clariant | | | | 30 | | | 30 | 30 | | | 30 |
| Rezal^{®} 67 = Summit^{®} AZ-7373 Powder INCl: Aluminum Zirconium Pentachlorohydrate Hersteller: Reheis. Inc. - jetzt Summit Research Labs | 15 | | | | 15 | 15 | | | | 15 | |
| Rezal^{®} 36 GC = Summit^{®} AZG-442 INCl: Aluminum Zirconium Tetrachlorohydrex Gly. Hersteller: Reheis. Inc. - jetzt Summit Research Labs | | 30 | 30 | | | | | | 30 | | |
| Hydagen^{®} C.A.T. INCl: Triethyl Citrate Hersteller: Cognis GmbH | | | | 2 | | | | | | 2 | |
| Irgasan INCl: Triclosan Hersteller: Ciba Corporation | 2 | | | | | | 3 | | | 1 | |
| Uliragel^{®} 300 INCl: Polyquatemium 37 Hersteller: Cognis GmbH | 3,5 | 5 | 4,5 | 3 | 3,5 | 2,5 | 2 | 4 | 3 | 4 | 3,5 |
| Ethanol | | | | 3 | | 2 | | | | | 3 |
| Butylenglykol | 2 | | | | | | 3 | | | 1 | |
| Glycerin | | 3 | | 5 | | | | | 7 4 | | |
| Wasser, Konservierungsmittel etc. | q.s. | q.s. | q.s. | q.s. | q.s. | q.s. | q.s. | q.s. | q.s. | q.s. | q.s. |

## Patentansprüche

1. Ethylenoxid-freie Antiperspirant/Desodorant-Zubereitungen, enthaltend:
a. Antiperspirant-/Desodorant-Wirkstoff,
b. ein quaternäres Polymer mit der INCl-Bezeichnung Polyquaternium 37.

2. Zubereitungen nach Anspruch 1, enthaltend:
a. Antiperspirant-/Desodorant-Wirkstoff,
b. ein quaternäres Polymer mit der INCl-Bezeichnung Polyquaternium 37, und
c. eine lipophile Phase, die aus flüssigem ÖI und/oder Wachs gebildet ist.

3. Zubereitungen nach Anspruch 1 oder 2, enthaltend:
a.1- 30 Gew.-% des Antiperspirant-/Desodorant-Wirkstoffs,
b. 0,1-10 Gew.-% des quaternären Polymers mit der INCl-Bezeichnung Polyquatemium 37, und
c.1- 20 Gew.-% der lipophilen Phase,
jeweils bezogen auf das Gesamtgewicht der Antiperspirant/Desodorant-Zubereitung.

4. Zubereitungen nach einem der vorhergehenden Ansprüche, enthaltend:
a. 5 - 20 Gew.-% des Antiperspirant-/Desodorant-Wirkstoffs,
b. 1- 5 Gew.-% des quaternären Polymers mit der INCl-Bezeichnung Polyquatemium 37, und
c. 5 - 15 Gew.-% der lipophilen Phase,
jeweils bezogen auf das Gesamtgewicht der Antiperspirant/Desodorant-Zubereitung.

5. Zubereitungen nach einem der vorhergehenden Ansprüche, enthaltend:
a. 10 -15 Gew.-% des Antiperspirant-/Desodorant-Wirkstoffs,
b. 1- 5 Gew.-% des quaternären Polymers mit der INCl-Bezeichnung Polyquaternium 37, und
c. 5 -15 Gew.-% der lipophilen Phase,
jeweils bezogen auf das Gesamtgewicht der Antiperspirant/Desodorant-Zubereitung.

6. Zubereitungen nach einem der vorhergehenden Ansprüche, **dadurch gekennzeichnet, dass** diese Emulgator-frei sind und eine lipophile Phase enthalten.

7. Zubereitungen nach einem der vorhergehenden Ansprüche in Form von Gelen oder Gelcremes.

8. Zubereitungen nach einem der vorhergehenden Ansprüche in Form von Antitranspirant-/Deosprays, Antitranspirant-/Deolotionen, Antitranspirant-/Deoroll-ons, Antitranspirant-/Deogelen, Antitranspirant-/Deocremes oder Antitranspirant-/Deostiften.

9. Verwendung von Polyquaternium 37 zur Herstellung von Ethylenoxid-freien kosmetischen oder pharmazeutischen Zubereitungen.

10. Verwendung von Polyquaternium 37 zur Herstellung von Emulgator-freien kosmetischen oder pharmazeutischen Zubereitungen.

11. Verwendung von Polyquaternium 37 zur Herstellung von Emulgator-freien, eine lipophile Phase enthaltenden kosmetischen oder pharmazeutischen Zubereitungen.

12. Verwendung nach einem der Ansprüche 9 bis 11 zur Herstellung von Antitranspirant-/Deosprays, Antitranspirant-/Deolotionen, Antitranspirant-/Deoroll-ons, Antitranspirant-/Deogelen, Antitranspirant-/Deocremes oder Antitranspirant-/Deostiften.
